(19) Europäisches Patentamt
European Patent Office
Office européen des brevets

(11) Veröffentlichungsnummer: **0 250 897**
**B1**

(12) **EUROPÄISCHE PATENTSCHRIFT**

(45) Veröffentlichungstag der Patentschrift: **23.05.90**

(51) Int. Cl.⁵: **C07C 251/48**
// C07D249/08

(21) Anmeldenummer: **87107949.7**

(22) Anmeldetag: **02.06.87**

(54) **Verfahren zur Herstellung von Hydroxybenzaldoxim-O-ethern.**

(30) Priorität: **14.06.86 DE 3620166**

(43) Veröffentlichungstag der Anmeldung:
**07.01.88 Patentblatt 88/1**

(45) Bekanntmachung des Hinweises auf die Patenterteilung:
**23.05.90 Patentblatt 90/21**

(84) Benannte Vertragsstaaten:
**BE CH DE FR GB IT LI**

(56) Entgegenhaltungen:
**EP-A- 0 023 890**
**EP-A- 0 083 010**
**EP-A- 0 121 701**
**EP-A- 0 158 159**
**EP-A- 0 218 123**
**EP-B- 0 076 370**
**DE-A- 2 927 117**

(73) Patentinhaber: **BAYER AG,**
**D-5090 Leverkusen 1 Bayerwerk(DE)**

(72) Erfinder: **Lantzsch, Reinhard, Dr., Am Buschhäusen 51, D-5600 Wuppertal 1(DE)**
Erfinder: **Elbe, Hans-Ludwig, Dr., Dasnöckel 59, D-5600 Wuppertal 11(DE)**
Erfinder: **Reiser, Wolf, Dr., Kiebitzweg 12a, D-5600 Wuppertal 1(DE)**
Erfinder: **Schmetzer, Johannes, Dr., Wacholderweg 45, D-5024 Pulheim(DE)**

**EP 0 250 897 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von bekannten Hydroxybenzaldoxim-O-ethern, die als Zwischenprodukte zur Synthese von Verbindungen mit fungizider, insektizider uund antimykotischer Wirksamkeit verwendet werden können.

Es ist bereits bekannt geworden, daß man bestimmte Hydroxybenzaldoxim-O-ether herstellen kann, indem man Hydroxybenzaldehyde mit den entsprechenden Hydroxylamin-Derivaten umsetzt (vgl. EP-A 0 076 370 und EP-A 0 115 828). Die betreffende Umsetzung läßt sich durch das folgende Reaktionsschema veranschaulichen:

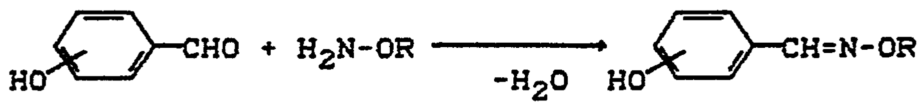


R = Alkyl, Alkenyl, Alkinyl.

Nachteilig an diesem Verfahren sind jedoch der hohe Preis und die schlechte Verfügbarkeit der als Reaktionskomponenten benötigten substituierten Hydroxylamine. Der Einsatz dieser Stoffe für eine Herstellung von Hydroxybenzaldoxim-O-ethern in technischem Maßstab ist daher problematisch.

Weiterhin ist bekannt, daß sich Oximether in Gegenwart von dipolar aprotischen Lösungsmitteln herstellen lassen (vg. DE-A 2 927 117). Die Verwendung von dipolaren, aprotischen Solventien im technischen Maßstab ist jedoch problematisch.

Schließlich ist auch bekannt, daß sich Oximether in Gegenwart von Alkoholen als Verdünnungsmittel synthetisieren lassen (vgl. EP-A 0 121 701). Nachteilig an diesem Verfahren ist aber, daß sich in Nebenreaktionen größere Mengen an Nitronen bilden und die Ausbeuten an den gewünschten Produkten relativ niedrig sind.

Es wurde nun gefunden, daß man bekannte Hydroxybenzaldoxim-O-ether der Formel

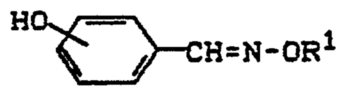 (I)

in welcher

$R^1$ für Alkyl steht,

erhält, wenn man tert.-Butoxy-benzaldoxime der Formel

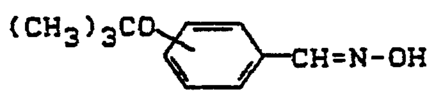 (II)

mit Alkylierungsmitteln der Formel

$R^1$–X (III)

in welcher

$R^1$ die oben angegebene Bedeutung hat und

X für Halogen oder $OSO_2OR^1$ steht,

wobei $R^1$ wiederum die oben angegebene Bedeutung hat,

in Gegenwart eines Alkalimetallhydroxids als starker Base sowie eines Tetraalkylammonium-Salzes oder Tetraalkylphosphonium-Salzes als Phasentransfer-Katalysator und in Gegenwart eines aromatischen Kohlenwasserstoffes oder chlorierten aromatischen Kohlenwasserstoffes als Verdünnungsmittel bei Temperaturen zwischen 0°C und 50°C umsetzt und anschließend bei Temperaturen zwischen 0°C und 50°C mit einer wasserfreien Säure aus der Gruppe bestehend aus Trifluoressigsäure, Methansulfonsäure, Perfluoralkancarbonsäure oder Halogenwasserstoff behandelt.

Es ist als überraschend zu bezeichnen, daß die erfindungsgemäße Umsetzung unter den angegebenen Verfahrensbedingungen glatt und in hervorragender Ausbeute abläuft. Aufgrund des bekannten Standes der Technik war nämlich damit zu rechnen, daß sich zumindest teilweise Produkte der Formel

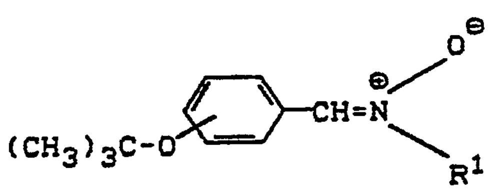 (IV)

bilden (vgl. EP-A 0 121 701). Unerwartet ist auch, daß die Abspaltung des tert.-Butyl-Restes in Abwesenheit von Wasser bereits unter sehr milden Bedingungen gelingt, so daß der Oximether-Rest im Verlauf

der Umsetzung nicht wieder zerstört wird, was bei Anwesenheit von Wasser zu befürchten wäre (vgl. EP-A 0 083 010).

Das erfindungsgemäße Verfahren zeichnet sich durch eine Reihe von Vorteilen aus. So ermöglicht es die Herstellung von Hydroxybenzaldoxim-O-ethern der Formel (I) in hervorragenden Ausbeuten, wobei gut zugängliche Verbindungen als Ausgangsprodukte eingesetzt werden. Günstig ist auch, daß nur solche Verdünnungsmittel erforderlich sind, deren Verwendung auch in technischem Maßstab problemlos ist. Außerdem braucht während der Durchführung des Verfahrens kein Wechsel der Lösungsmittels vorgenommen zu werden. Ferner ist die Umsetzung bei niedrigen Temperaturen einfach durchführbar, und die Isolierung der Hydroxybenzaldoxim-O-ether der Formel (I) bereitet keinerlei Schwierigkeiten. Das erfindungsgemäße Verfahren ist daher besonders geeignet, um Hydroxybenzaldoxim-O-ether auch in technischem Maßstab herzustellen.

Verwendet man beispielsweise 4-tert.-Butoxybenzaldoxim als Ausgangsstoff, Diethylsulfat als Alkylierungsmittel, Kaliumhydroxid als starke Base, Tetrabutyl-ammoniumbromid als Phasentransfer-katalysator, Toluol als Verdünnungsmittel und Trifluoressigsäure als Säure, so kann der Verlauf des erfindungsgemäßen Verfahrens durch das folgende Formelschema veranschaulicht werden:

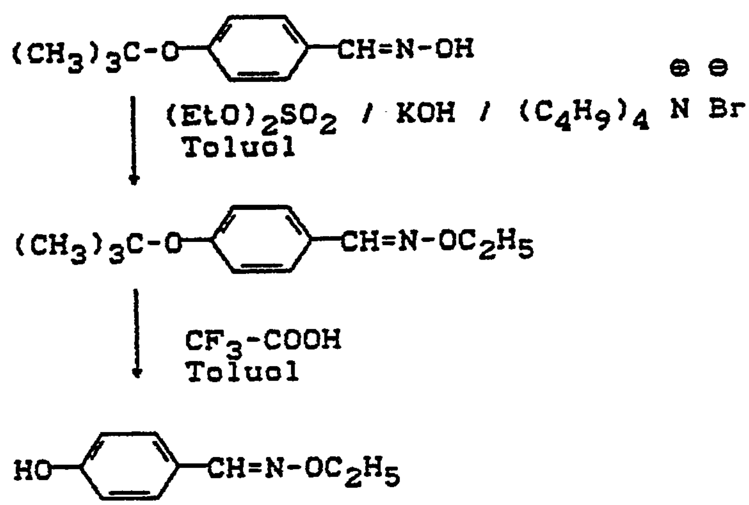


Die bei der Durchführung des erfindungsgemäßen Verfahrens als Ausgangsstoffe benötigten tert.-Butoxy-benzaldoxime sind durch die Formel (II) definiert. Als Beispiele für diese Stoffe seien 4-tert.-Butoxy-benzaldoxim, 3-tert.-Butoxy-benzaldoxim und 2-tert-Butoxy-benzaldoxim genannt.

Die tert.-Butoxy-benzaldoxime der Formel (II) snd bisher noch nicht bekannt. Sie lassen sich herstellen, indem man Aldehyde der Formel

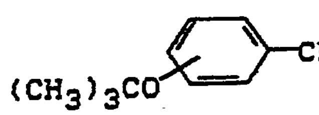 (V)

oder deren Acetale mit Hydroxylamin oder Hydroxylamin-Salzen in Gegenwart eines Verdünnungsmittels sowie gegebenenfalls in Gegenwart eines Katalysators umsetzt.

Die Aldehyde der Formel (V) und deren Acetale sind bekannt oder lassen sich nach prinzipiell bekannten Verfahren in einfacher Weise herstellen. So erhält man Aldehyde der Formel (V), indem man Verbindungen der Formel

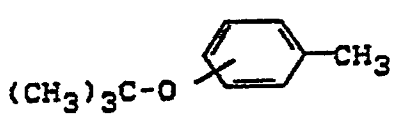 (VI)

elektrochemisch oxidiert.

Die bei dem obigen Verfahren zur Herstellung von tert.-Butoxy-benzaldoximen der Formel (II) weiterhin als Ausgangsstoffe benötigten Hydroxylamin-Salze und das Hydroxylamin sind bekannt. Als Beispiele seien Hydroxylamin-Hydrochlorid unf Hydroxylamin-Sulfat genannt.

Als Verdünnungsmittel können bei dem obigen Verfahren zur Herstellung von tert.-Butoxy-benzaldoximen der Formel (II) alle für derartige Umsetzungen üblichen Solventien verwendet werden. Vorzugsweise in Betracht kommen Alkohole, wie Methanol, Ethanol, Propanol und Butanol, oder auch Gemische aus Alkoholen und Wasser.

Als Katalysatoren kommen bei dem obigen Verfahren zur Herstellung von tert.-Butoxy-benzaldoximen

der Formel (II) alle für derartige Umsetzungen üblichen Reaktionsbeschleuniger in Frage. Vorzugsweise verwendbar sind Salze mit Pufferwirkung, wie zum Beispiel Natriumacetat, und ferner schwache Säuren, wie zum Beispiel Essigsäure.

Die Reaktionstemperaturen können bei dem Verfahren zur Herstellung der tert.-Butoxy-benzaldoxime der Formel (II) innerhalb eines bestimmten Bereiches variiert werden. Im allgemeinen arbeitet man bei Temperaturen zwischen 0°C und 50°C, vorzugsweise zwischen 20°C und 40°C.

Bei der Durchführung des obigen Verfahrens zur Herstellung der tert.-Butoxy-benzaldoxime der Formel (II) setzt man Aldehyde der Formel (V) oder auch deren Acetale mit einer äquivalenten Menge oder auch einem Überschuß an Hydroxylamin oder Hydroxylamin-Salzen, gegebenenfalls in Gegenwart eines Katalysators um. Die Ausarbeitung erfolgt nach üblichen Methoden.

Die bei dem erfindungsgemäßen Verfahren als Reaktionskomponenten benötigten Alkylierungsmittel sind durch die Formel (III) allgemein definiert. In dieser Formel steht $R^1$ vorzugsweise für Alkyl mit 1 bis 10 Kohlenstoffatomen, und X steht vorzugsweise für Chlor, Brom, Iod oder den Rest der Formel -$OSO_2OR^1$, wobei $R^1$ wiederum vorzugsweise für Alkyl mit 1 bis 10 Kohlenstoffatomen steht.

Besonders bevorzugt sind diejenigen Stoffe der Formel (III), in denen $R^1$ für Alkyl mit 1 bis 4 Kohlenstoffatomen steht und X für Chlor, Brom oder den Rest der Formel $-OSO_2OR^1$ steht, wobei $R^1$ wiederum besonders bevorzugt für Alkyl mit 1 bis 4 Kohlenstoffatomen steht.

Als Beispiele für Alkylierungsmittel der Formel (III) seien genannt:

Methylchlorid, Methylbromid, Dimethylsulfat, Ethylchlorid, Ethylbromid und Diethylsulfat.

Die Alkylierungsmittel der Formel (III) sind bekannt.

Als starke Basen kommen bei der Durchführung des erfindungsgemäßen Verfahrens Alkalimetallhydroxide, wie Natriumhydroxid oder Kaliumhydroxid in Betracht.

Als Phasentransfer-Katalysatoren kommen bei der Durchführung des erfindungsgemäßen Verfahrens Tetraalkyl-ammonium-Salze oder Tetraalkyl-phosphonium-Salze, wie zum Beispiel Tetrabutyl-ammonium-bromid, in Frage.

Als Verdünnungsmittel kommen bei der Durchführung des erfindungsgemäßen Verfahrens aromatische Kohlenwasserstoffe oder chlorierte aromatische Kohlenwasserstoffe in Betracht. Beispielhaft genannt seien Toluol, Xylole, Chlorbenzol oder ortho-Dichlorbenzol.

Als wasserfreie Säuren kommen bei der Durchführung des erfindungsgemäßen Verfahrens Trifluoressigsäure, Methansulfonsäure, Perfluoralkansulfonsäuren und Halogenwasserstoffe, wie Chlorwasserstoff und Bromwasserstoff, in Frage.

Die Reaktionstemperaturen können bei der Durchführung des erfindungsgemäßen Verfahrens in einem bestimmten Bereich variiert werden. Im allgemeinen arbeitet man sowohl bei der eigentlichen Umsetzung als auch bei der Behandlung mit Säure bei Temperaturen zwischen 0°C und 50°C, vorzugsweise zwischen 10°C und 30°C.

Die erfindungsgemäße Umsetzung wird im allgemeinen unter Normaldruck vorgenommen. Es ist aber auch möglich, unter erhöhtem oder vermindertem Druck zu arbeiten.

Bei der Durchführung des erfindungsgemäßen Verfahrens setzt man auf 1 Mol an tert.-Butoxy-benzaldoxim der Formel (II) im allgemeinen 1 bis 2 Mol, vorzugsweise 1 bis 1,5 Mol an Alkylierungsmittel der Formel (III) sowie 1 bis 3 Mol, vorzugsweise 2,1 bis 2,5 Mol Base sowie gegebenenfalls eine katalytische Menge eines Phasentransfer-Katalysators ein. Für die anschließende Säurebehandlung genügen katalytische Mengen an Säure; es kann jedoch auch ein Überschuß an Säure eingesetzt werden. Findet eine Salzbildung statt, so müßten mindestens äquimolare Säuremengen verwendet werden.

Im allgemeinen geht man bei der Durchführung des erfindungsgemäßen Verfahrens so vor, daß man das nach der ersten Umsetzung anfallende Reaktionsgemisch durch Filtration von enthaltenen Salzen befreit und den entstandenen tert.-Butoxy-benzaldoxim-O-ester entweder nach vorheriger Isolierung oder direkt in Gegenwart eines Verdünnungsmittels mit Säure behandelt. Die anschließende Aufarbeitung erfolgt nach üblichen Methoden. So kann man zum Beispiel in der Weise verfahren, daß man das Reaktionsgemisch mit Wasser versetzt, die organische Phase abtrennt, nacheinander mit wäßrig-basischer Lösung und Wasser wäscht und nach dem Trocknen einengt. Wenn die gewünschten Produkte der Formel (I) in Form von Salzen anfallen, so können diese aus dem Gemisch abfiltriert werden und durch Behandlung mit wäßrig-basischer Lösung in die freien Hydroxy-benzaldoxim-O-ether der Formel (I) überführt werden.

Die nach dem erfindungsgemäßen Verfahren herstellbaren Hydroxybenzaldoxim-O-ether der Formel (I) sind allgemein bekannte Ausgangsstoffe zur Synthese von biologisch aktiven Verbindungen, wie z.B. zur Synthese von Oximethern, welche gute insektizide Eigenschaften besitzen (vgl. EP-A 0 115 828); von Azolyl-phenoxy-Derivaten, welche hervorragende fungizide Eigenschaften aufweisen (vgl. EP-A 0 076 370); von 1-Hydroxyethyl-triazolyl-Derivaten, welche gute fungizide und antimykotische Eigenschaften aufweisen (vgl. EP-A 0 110 048 und DE-A 3 314 548); sowie von Hydroxyalkyl-azol-Derivaten, welche gute antimykotische Wirksamkeit besitzen (vgl. DE-A 3 427 844).

So läßt sich beispielsweise das 3,3-Dimethyl-1-(4-methoximinomethyl-phenoxy)-1-(1,2,4-triazol-1-yl)-butan-2-on der Formel

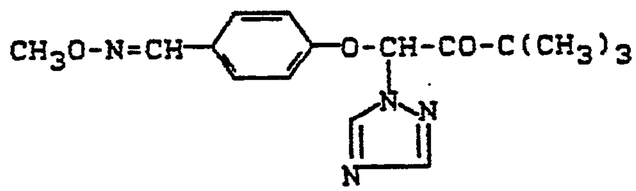

herstellen, indem man 3,3-Dimethyl-1-(1,2,4-triazol-1-yl)-butan-2-on zunächst mit Brom zum 1-Brom-(81,2,4-triazol-1-yl)-3,3-dimethyl-butan-2-on umsetzt und dieses anschliessend mit 4-Hydroxybenzaldehyd-O-methyl-oximether in Gegenwart einer Base umsetzt. Diese Synthese läßt sich formelmäßig wie folgt veranschaulichen:

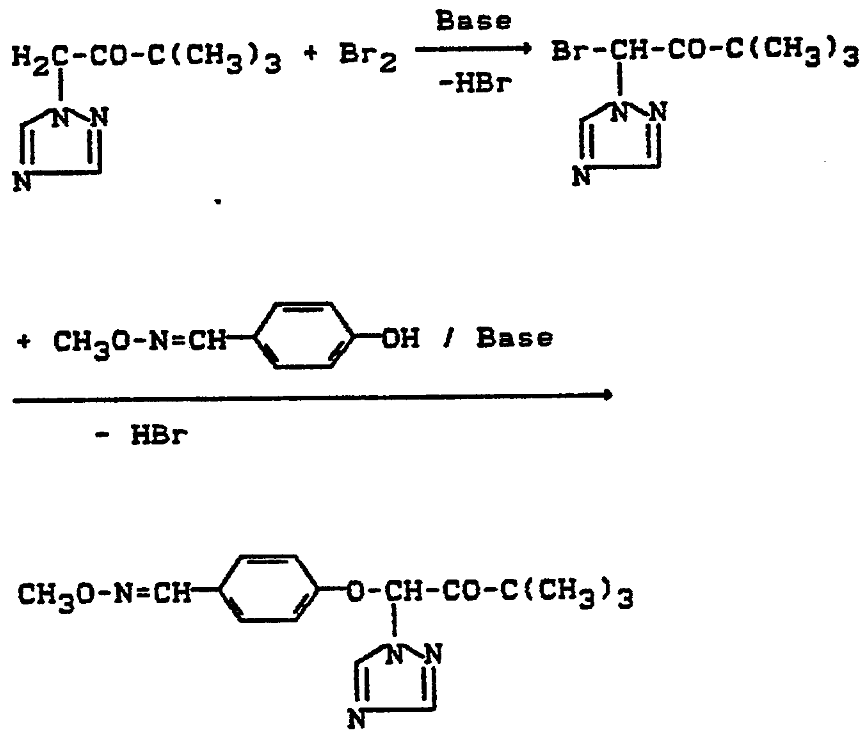


Das erfindungsgemäße Verfahren wird durch das nachfolgende Beispiel veranschaulicht.

<u>Beispiel 1</u>

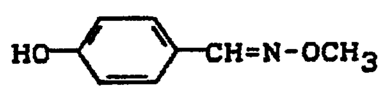

In eine Lösung von 48 g eines Produktes, das zu 90,2 Gew.-% aus 4-tert.-Butoxy-benzaldoxim (= 0,224 Mol) besteht, und 2 g Tetrabutylammonium-bromid in 500 ml Toluol werden bei Raumtemperatur 14 g (0,25 Mol) Kaliumhydroxid gegeben. Man rührt noch 15 Minuten bei 20 - 25°C, läßt dann bei dieser Temperatur 35 g (0,228 Mol) Dimethylsulfat zutropfen und rührt 12 Stunden nach. Anschließend werden die entstandenen Salze abfiltriert und es wird ein Überschuß an Chlorwasserstoff in das Reaktionsgemisch eingeleitet. Danach wird 4 Stunden bei 20 - 25°C gerührt und filtriert. Man erhält auf diese Weise 38,7 g (92 % der Theorie) an 4-Hydroxy-benzaldoxim-O-methylether in Form des Hydrochlorids.
Fp. 148°C

Das Hydrochlorid wird durch Behandeln mit wäßriger Natriumbicarbonat-Lösung in den 4-Hydroxy-benzaldoxim-O-methylether überführt.
Ausbeute: quantitativ
Kp. 116°C/0,05 mbar

Herstellung des Ausgangsproduktes:

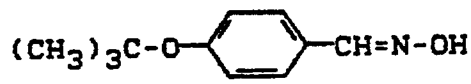

In eine Lösung von 38,3 g Hydroxylamin-Hydrochlorid und 45 g Natriumacetat in 700 ml Methanol und 35 ml Wasser werden 89 g 4-tert.-Butoxy-benzaldehyd unter Rühren eingetropft. Nach dem Abklingen der leicht exothermen Reaktion rührt man 12 Stunden bei 20 - 25°C nach, filtriert und engt das Filtrat unter vermindertem Druck ein. Der Rückstand wird in Methylenchlorid aufgenommen und die entstehende organische Phase wird nacheinander mit wäßriger Natriumhydrogencarbonat-Lösung und Wasser gewaschen. Nach dem Trocknen und Einengen unter vermindertem Druck verbleiben 95,8 g einer kristallinen Substanz vom Fp. 70-75°C. Gemäß Gaschromatogramm besteht das Produkt zu 90,2 Gew.-% aus 4-tert.-Butoxy-benzaldoxim (syn + anti). Die Ausbeute errechnet sich danach zu 89,5 % der Theorie.

<u>Beispiel 2</u>

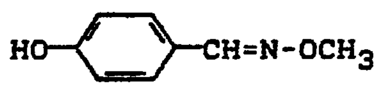

20,7 g (0,1 Mol) 4-tert.-Butoxy-benzaldoxim-O-methylether werden in 200 ml Toluol gelöst und mit 20 ml Trifluoressigsäure versetzt. Mann rührt 12 Stunden bei Raumtemperatur, gießt dann das Reaktionsgemisch in Wasser und trennt die organische Phase ab. Die wäßrige Phase wird noch dreimal mit Methylenchlorid extrahiert. Die vereinigten organischen Phasen werden nacheinander mit wäßriger Natriumhydrogencarbonat-Lösung und Wasser gewaschen, dann getrocknet und unter vermindertem Druck eingeengt. Man erhält auf diese Weise 13 g (86 % der Theorie) an 4-Hydroxy-benzaldoxim-O-methylether.
Kp. 112-114°C/0,05 mbar

<u>Beispiel 3</u>

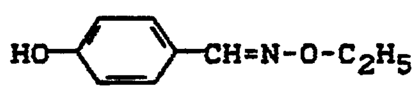

Nach der im Beispiel 1 beschriebenen Methode wird 4-tert.-Butoxy-benzaldoxim mit Diethylsulfat umgesetzt. Die Aufarbeitung erfolgt ebenfalls nach der im Beispiel 1 angegebenen Methode. Man erhält auf diese Weise 4-Hydroxy-benzaldoxim-O-ethylether in Form einer Festsubstanz vom Schmelzpunkt 64°C.

<u>Beispiel 4</u>

<u>Herstellung der Verbindung der Formel:</u>

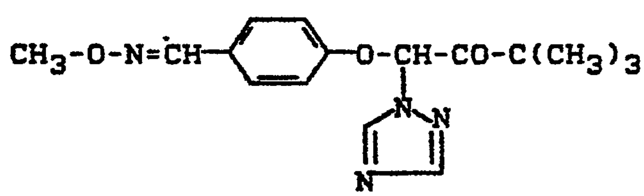

In eine Lösung von 217 g (1,3 Mol) 3,3-Dimethyl-1-(1,2,4-triazol-1-yl)-2-butanon in 700 ml Eisessig trägt man 110 g (1,34 Mol) Natriumacetat ein, wobei die Temperatur auf ca. 28°C ansteigt. Man läßt 30 Minuten nachrühren und versetzt tropfenweise unter leichter Kühlung bei 30 bis 33°C mit 208 g (1,3 Mol) Brom. Das Reaktionsgemisch wird 2,5 Stunden bei Raumtemperatur nachgerührt und in 1200 ml Wasser gegeben. Man extrahiert mit Methylenchlorid, wäscht mit Wasser und wäßriger Bicarbonatlösung, trocknet über Natriumsulfat und engt ein.

Das so erhaltene rohe 1-Brom-3,3-Dimethyl-1-(1,2,4-triazol-1-yl)-2-butanon wird in 100 ml Acetonitril gelöst und zu einer Aufschlämmung von 151 g (1 Mol) 4-Hydroxy-benzaldoxim-O-methylether und 150 g (1,09 Mol) Kaliumcarbonat in 800 ml Acetonitril gegeben, wobei die Temperatur auf etwa 40°C ansteigt. Man läßt das Reaktionsgemisch 3 Stunden bei 60 bis 65°C nachrühren, kühlt anschließend ab und gibt auf Wasser. Man extrahiert mit Toluol, wäscht mit Wasser, trocknet und engt ein. Der Rückstand wird in

Ligroin verrieben und auf Ton getrocknet. Man erhält 241 g (76 % der Theorie) 3,3-Dimethyl-1-(4-methoximinomethyl-phenoxy)-1-(1,2,4-triazol-1-yl)-butan-2-on vom Schmelzpunkt 83-87°C.

## Patentansprüche

1. Verfahren zur Herstellung von Hydroxybenzaldoxim-O-ethern der Formel

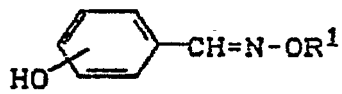 (I)

in welcher
$R^1$ für Alkyl steht,
dadurch gekennzeichnet, daß man tert.-Butoxy-benzaldoxime der Formel

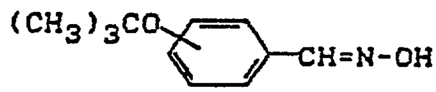 (II)

mit Alkylierungsmitteln der Formel
$R^1$–X (III)
in welcher
$R^1$ die oben angegebene Bedeutung hat und
X für Halogen oder $OSO_2OR^1$ steht,
wobei $R^1$ wiederum die oben angegebene Bedeutung hat,
in Gegenwart eines Alkalimetallhydroxids als starker Base sowie eines Tetraalkylammonium-Salzes oder Tetraalkyl-phosphonium-Salzes als Phasentransfer-Katalysator und in Gegenwart eines aromatischen Kohlenwasserstoffes oder chlorierten atomatischen Kohlenwasserstoffes als Verdünnungsmittel bei Temperaturen zwischen 0°C und 50°C umsetzt und anschließend bei Temperaturen zwischen 0°C und 50°C mit einer wasserfreien Säure aus der Gruppe bestehend aus Trifluoressigsäure, Methansulfonsäure, Perfluoralkancarbonsäure oder Halogenwasserstoff behandelt.

2. Verfahren gemäß Anspruch 1, dadurch gekennzeichnet, daß man 4-tert.-Butoxy-benzaldoxim als Ausgangsstoff der Formel (II) einsetzt.

3. Verfahren gemäß Anspruch 1, dadurch gekennzeichnet, daß man Alkylierungsmittel der Formel (III) einsetzt, in denen
$R^1$ für Alkyl mit 1 bis 10 Kohlenstoffatomen steht und
X für Chlor, Brom, Iod oder den Rest der Formel $-OSO_2OR^1$ steht, wobei $R^1$ wiederum für Alkyl mit 1 bis 10 Kohlenstoffatomen steht.

4. Verfahren gemäß Anspruch 1, dadurch gekennzeichnet, daß man bei Temperaturen zwischen 10°C und 30°C arbeitet.

5. Tert.-Butoxy-benzaldoxime der Formel

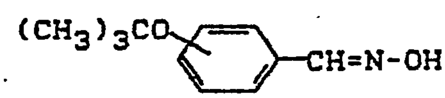 (II).

6. Verfahren zur Herstellung von tert.-Butoxy-benzaldoximen der Formel

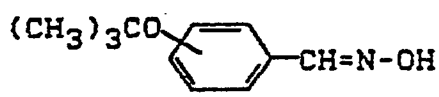 (II)

dadurch gekennzeichnet, daß man Aldehyde der Formel

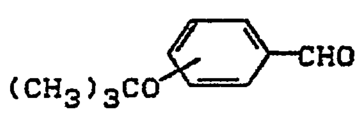 (V)

oder deren Acetale mit Hydroxylamin oder Hydroxylamin-Salzen in Gegenwart eines Verdünnungsmittels sowie gegebenenfalls in Gegenwart eines Katalysators umsetzt.

## Claims

1. Process for the preparation of hydroxybenzaldoxime O-ethers of the formula

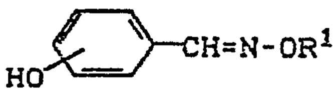 (I)

in which

$R^1$ represents alkyl,

characterized in that tert.-butoxy-benzaldoximes of the formula

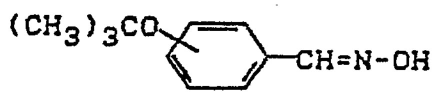 (II)

are reacted with alkylating agents of the formula

$R^1$–X (III)

in which

$R^1$ has the abovementioned meaning and

X represents halogen or $OSO_2OR^1$,

wherein

$R^1$ again has the abovementioned meaning,

in the presence of an alkali metal hydroxide as strong base and of a tetraalkylammonium salt or tetraalkylphosphonium salt as phase transfer catalyst and in the presence of an aromatic hydrocarbon or chlorinated aromatic hydrocarbon as diluent at temperatures between 0°C and 50°C, and the products are then treated with an anhydrous acid from the group consisting or trifluoroacetic acid, methanesulphonic acid, perfluoroalkanecarboxylic acid or hydrogen halide at temperatures between 0°C and 50°C.

2. Process according to Claim 1, characterized in that 4-tert.-butoxy-benzaldoxime is employed as the starting substance of the formula (II).

3. Process according to Claim 1, characterized in that alkylating agents of the formula (III) in which

$R^1$ represents alkyl with 1 to 10 carbon atoms and

X represents chlorine, bromine, iodine or the radical of the formula $-OSO_2OR^1$,

wherein

$R^1$ again represent alkyl with 1 to 10 carbon atoms,

are employed.

4. Process according to Claim 1, characterized in that it is carried out at temperatures between 10°C and 30°C.

5. Tert.-butoxy-benzaldoximes of the formula

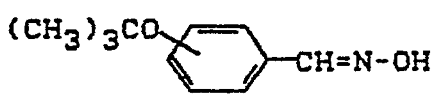 (II).

6. Process for the preparation of tert.-butoxybenzaldoximes of the formula

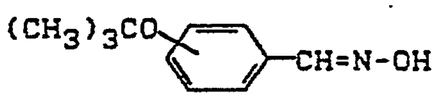 (II)

characterized in that aldehydes of the formula

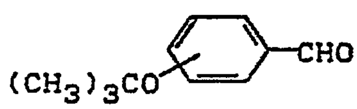 (V)

or acetals thereof are reacted with hydroxylamine or hydroxylamine salts in the presence of a diluent and, if appropriate, in the presence of a catalyst.

**Revendications**

1. Procédé de production d'hydroxybenzaldoxime-O-éthers de formule

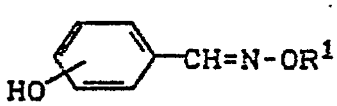 (I)

dans laquelle

$R^1$ est un groupe alkyle,
caractérisé en ce qu'on fait réagir des tertiobutoxybenzaldoximes de formule

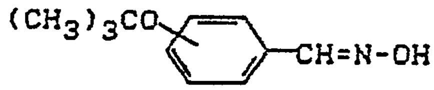 (II)

avec des agents d'alkylation de formule
$R^1$–X (III)
dans laquelle
$R^1$ a la définition indiquée ci-dessus et
X est un halogène ou un groupe $OSO_2OR^1$,
dans lequel $R^1$ a, là encore, la définition indiquée ci-dessus,
en présence d'un hydroxyde de métal alcalin comme base forte ainsi que d'un sel de tétra-alkylammonium ou d'un sel de tétra-alkylphosphonium comme catalyseur de transfert de phase et en présence d'un hydrocarbure aromatique ou d'un hydrocarbure aromatique chloré comme diluant, à des températures comprises entre 0°C et 50°C, puis on traite le produit à des températures comprises entre 0°C et 50°C avec un acide anhydre choisi dans le groupe comprenant l'acide trifluoracétique, l'acide méthansulfonique, un acide perfluoralcanecarboxylique ou un halogénure d'hydrogène.

2. Procédé suivant la revendication 1, caractérisé en ce qu'on utilise comme composé de départ de formule (II) la 4-tertiobutoxybenzaldoxime.

3. Procédé suivant la revendication 1, caractérisé en ce qu'on utilise des agents d'alkylation de formule (III) dans lequels
$R^1$ est un groupe alkyle ayant 1 à 10 atomes de carbone et
X représent le chlore, le brome, l'iode ou le reste de formule $-OSO_2OR^1$ dans laquelle $R^1$ représente, là encore, un groupe alkyle ayant 1 à 10 atomes de carbone.

4. Procédé suivant la revendication 1, caractérisé en ce qu'on opère à des températures comprises entre 10 et 30°C.

5. Des tertiobutoxybenzaldoximes de formule

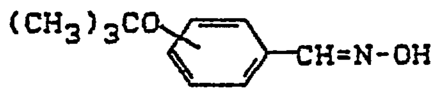 (II).

6. Procédé de production de tertiobutoxybenzaldoximes de formule

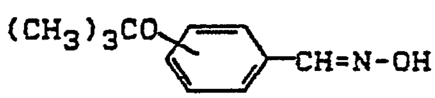 (II)

caractérisé en ce qu'on fait réagir des aldéhydes de formule

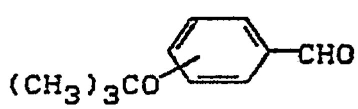 (V)

ou leurs acétals avec l'hydroxylamine ou des sels d'hydroxylamine en présence d'un diluant ainsi que, le cas échéant, en présence d'un catalyseur.